# EUROPEAN PATENT APPLICATION

(11) **EP 1 839 501 A1**
(43) Date of publication of application: **03.10.2007**
(21) Application number: 05806950.1
(22) Date of filing: 17.11.2005
(51) Int. Cl.: A23L 1/30, A23L 2/52

(54) **SITOSTEROL COMPOUND-CONTAINING COMPOSITION AND PROCESS FOR PRODUCING THE SAME**

(30) Priority: 17.11.2004 JP 2004333503
(71) Applicant: SAN-EI GEN F.F.I., INC., Toyonaka-shi, Osaka 561-8588 (JP)
(72) Inventor: ORIKOSHI, Hideyuki, Toyonaka-shi, Osaka 5618588 (JP); OKUDA, Takahide, Toyonaka-shi, Osaka 5618588 (JP)
(74) Representative: Müller-Boré & Partner Patentanwälte
(86) International application number: PCT/JP2005/021101
(87) International publication number: WO 2006/054627

(57) **Abstract**

A method for producing a phytosterol/phytostanol-containing composition is provided that facilitates dispersion in water of a phytosterol and/or phytostanol (phytosterol/phytostanol), which are difficult to dissolve in water, inhibits the occurrence of precipitation and coagulation (ring formation) for a long period of time even when incorporated in water, and can maintain a stable dispersed state. The method of the present invention has at least one of the following steps (1) to (3): (1) mixing at least one type selected from a pulverized phytosterol and phytostanol with an aqueous raw material containing an emulsifier having an HLB value of 6 to 16; (2) pulverizing at least one type selected from a phytosterol and a phytostanol followed by mixing with an aqueous raw material containing an emulsifier having an HLB value of 6 to 16; and (3) mixing at least one type selected from a phytosterol and a phytostanol with an aqueous raw material containing an emulsifier having an HLB value of 6 to 16, and pulverizing the phytosterol and/or phytostanol in the mixture.

## Description

### TECHNICAL FIELD

The present invention relates to a method for producing a composition having superior aqueous dispersion stability containing at least one type selected from the group consisting of a phytosterol and a phytostanol (also referred to as a "phytosterol/phytostanol -containing composition" in the present description). More particularly, the present invention relates to a method for producing a phytosterol/phytostanol-containing composition capable of maintaining a homogeneous and stable dispersed state over a long period of time when incorporated in water by significantly inhibiting coagulation or precipitation of the phytosterol or phytostanol. In addition, the present invention relates to a phytosterol/phytostanol-containing composition obtained according to this method, and to a food containing this phytosterol/phytostanol-containing composition. Moreover, the present invention relates to a method for stably dispersing a phytosterol or phytostanol in an aqueous liquid product.

### BACKGROUND ART

Excessive ingestion of fats and cholesterol contained in foods is a factor that increases blood cholesterol levels leading to diseases such as hyperlipemia, arteriosclerosis, arrhythmia and myocardial infarction. Therefore, attention is being focused on phytosterol as a substance that lowers cholesterol levels in the blood by inhibiting absorption of cholesterol into the body.

Phytosterol is the generic term for various sterols and mixtures thereof contained in plants. Known specific examples of phytosterols include β-sitosterol, stigmasterol, campesterol and brassicasterol. Phytosterols have the action of lowering blood cholesterol levels, and oral ingestion of phytosterols and esters thereof has been clearly demonstrated to remarkably lower blood cholesterol levels (Non-patent document 1: Peterson, et al., J. Nutir., 50, 1991 (1995)).

Consequently, foods, and particularly easily ingestible foods, containing phytosterols having the above-mentioned superior physiological action are useful from the standpoint of promoting health.

However, since phytosterols are oily substances, their solubility in water is extremely low, and since they also have highly crystalline structure, they have a high melting point (120 to 150°C) . Consequently, they are susceptible to precipitation and ring formation in water due to crystallization and coagulation, thereby making it extremely difficult to obtain these substances in the form of a stable liquid by uniformly dispersing in water.

In general, oily components can be stably dispersed in an aqueous medium by using a surfactant . However, in the case of phytosterols, it is difficult to stably disperse in an aqueous medium by using a surfactant alone, and other methods are required for improving dispersibility and solubility particularly with respect to water.

Several technologies have been proposed for dissolving phytosterols in water. Examples of such technologies include a method in which an oily phase, incorporating a phytosterol, lipophilic emulsifier such as a glycerin fatty acid ester and edible oil such as a medium-chain fatty acid, and an aqueous phase, comprised of a hydrophilic polyglycerin fatty acid ester and water, are uniformly mixed so that the HLB value of the emulsifier in the entire mixture is 12 or more (Patent-document 1: Unexamined Japanese Patent PublicationNo. 2002-291442), a method in which a sterol, a lipophilic emulsifier having an HLB of less than 8 and a dispersant such as an oil that is a liquid at room temperature are blended at a specific ratio (Patent-document 2: Examined Japanese Patent Publication No. 1994-59164), and a method in which a phytosterol, lecithin, oil, polyvalent alcohol and ethanol are blended at a specific ratio to obtain an oil-in-water emulsified composition (Patent-document 3: Unexamined Japanese Patent Publication No. 2001-117).

In general, the state in which a liquid dispersate is finely dispersed in a dispersion medium is an emulsion, and technologies for finely dispersing a liquid oily dispersate such as an oil, oily fragrance, oily vitamin or oily pigment in an aqueous dispersion medium are widely used in fields such as foods, pharmaceuticals and cosmetics.

Methods for stabilizing phytosterols in aqueous media by using this emulsification technology have also been studied. Examples of these methods that have been previously disclosed include a method in which a sterol is dispersed in a viscous vehicle and adjusted to a size of 10 µm or less using homogenization or other mechanical impact force (Patent-document 4: Japanese Published Translation No. 2002-535975 of PCT International Publication), a method in which a sterol is extracted by supercritical extraction to a particle diameter of 10 to 300 nm followed by stabilizing the particles with gelatin or chitosan protective colloid (Patent-document 5: Japanese Published Translation No. 2003-516115 of PCT International Publication), a method in which a phytosterol and cyclodextrin and so on are obtained in the form of an inclusion compound (Patent-document 6: Japanese Published Translation No. 2002-517418 of PCT International Publication), a method in which the use of a sterol and a sucrose fatty acid ester are combined to form a lamellar structure (Patent-document 7: Unexamined Japanese Patent Publication No. 1998-231229), and a method in which a phytosterol and at least one emulsifier selected from a sucrose fatty acid ester, sorbitan fatty acid ester and polyglycerin fatty acid ester are melted by heating at 60 to 200°C followed by rapidly stirring the resulting aqueous solvent (Patent-document 8: Japanese Published Translation No. 2004-510420 of PCT International Publication). However, these technologies still have room for improvement with respect to improving the dispersibility and solubility of phytosterols in aqueous media. In addition, since the method described in the Patent-document 8 requires a large amount of emulsifier in order to obtain adequate dispersibility and solubility for the phytosterol, it has the problem of impairing the flavor of foods when added to foods, thereby making it difficult to actually apply to foods.

### DISCLOSURE OF THE INVENTION

Since phytosterols are components that are difficult to dissolve in water as previously described, it is difficult to prepare a stable composition by incorporating in a food containing water. Thus, in order to produce a food containing a phytosterol in a stable state, it is necessary to employ a composition that allows a phytosterol to be easily dispersed in water and is capable of stably maintaining a dispersed state even after having been dispersed.

Therefore, an object of the present invention is to provide a method for producing a composition that allows a phytosterol or a hydration or hydrogenation product thereof in the form of a phytostanol to be easily dispersed in water and is capable of stably maintaining a dispersed state even after having been dispersed. In addition, an object of the present invention is to provide a composition containing a phytosterol or phytostanol obtained according to this method (phytosterol/phytostanol-containing composition) and a food containing this composition in a stable state.

Moreover, an object of the present invention is to provide a method for stably dispersing a phytosterol or a phytostanol in an aqueous liquid product.

As a result of conducting extensive studies for solving the above-mentioned problems, the inventors of the present invention found that a composition prepared by adding a fine phytosterol or a phytostanol obtained by pulverization treatment to an aqueous raw material containing an emulsifier having an HLB value of 6 to 16, or by finely pulverizing a phytosterol or a phytostanol in the above-mentioned aqueous raw material, facilitates dispersion in water and is capable of stably maintaining dispersion even after having been dispersed without causing problems such as coagulation or precipitation, thereby confirming that this composition is a composition having the desired characteristics as described above. The present invention was completed on the basis of this finding.

### I. Production Method of Phytosterol/phytostanol-Containing Composition

The present invention first provides a method for producing a composition containing a phytosterol or a phytostanol that solves the above-mentioned problems. The present invention includes the aspects indicated below.
Item 1. A method for producing a composition containing at least one type selected from a phytosterol and a phytostanol, comprising at least one of the following steps (1) to (3):
   (1) mixing at least one type selected from a pulverized phytosterol and phytostanol with an aqueous raw material containing an emulsifier having an HLB value of 6 to 16;
   (2) pulverizing at least one type selected from a phytosterol and a phytostanol followed by mixing with an aqueous raw material containing an emulsifier having an HLB value of 6 to 16; and
   (3) mixing at least one type selected from a phytosterol and a phytostanol with an aqueous raw material containing an emulsifier having an HLB value of 6 to 16, and pulverizing the phytosterol and/or phytostanol in the mixture.
Item 2. The production method according to Item 1, wherein the mean particle diameter of the pulverized phytosterol and/or phytostanol is 10 µm or less. Furthermore, in the case the composition of 1 contains both a phytosterol and a phytostanol, the mean particle diameters of the phytosterol and the phytostanol are both 10 µm or less.
Item 3. The production method according to Item 1 or 2, wherein the composition containing at least one type selected from a phytosterol and a phytostanol contains the phytosterol or the phytostanol (the total amount thereof in the case both are contained) at a ratio of 0.05 to 90% by weight.
Item 4. The production method according to any one of Items 1 to 3, wherein the emulsifier is at least one type selected from a sucrose fatty acid ester, a polyglycerin fatty acid ester and a sorbitan fatty acid ester having an HLB value of 10 or more.
Item 5. The production method according to any one of Items 1 to 4, wherein the composition containing at least one type selected from a phytosterol and a phytostanol contains an emulsifier at a ratio of 0.01 to 50% by weight.
Item 6. The production method according to any one of Items 1 to 5, wherein the composition containing at least one type selected from a phytosterol and a phytostanol contains the phytosterol or the phytostanol (the total amount thereof in the case both are contained) and an emulsifier at a weight ratio of 1:10 to 100:1.

### II. Phytosterol/phytostanol-Containing Composition

In addition, the present invention provides a composition obtained by the production method described in each of the above Items that contains at least one type selected from a phytosterol and a phytostanol and has superior aqueous dispersibility. This invention includes the aspects indicated below.
Item 7. A composition containing at least one type selected from a phytosterol and a phytostanol obtained by the production method described in any one of Items 1 to 6.
Item 8. The composition according to Item 7, wherein the mean particle diameter of the phytosterol and/or phytostanol is 10 µm or less. Furthermore, in the case the composition of 7 contains both a phytosterol and a phytostanol, the mean particle diameters of the phytosterol and the phytostanol are both 10 µm or less.

### III.Food Containing a Phytosterol/phytostanol-Containing Composition and Production Method of the Same

Moreover, the present invention provides a food stably containing a phytosterol and/or a phytostanol as a result of being produced using the above-mentioned phytosterol/phytostanol-containing composition having superior aqueous dispersibility. This invention includes the following inventions.
Item 9. A food containing the composition described in Item 7 or 8.
Item 10. The food according to Item 9, wherein the composition described in Item 7 or 8 is contained at the ratio of 0.1 to 3.2% by weight as the amount of the phytosterol or the phytostanol (the total amount thereof in the case both are contained).
Item 11. The food according to Item 9 or 10, which is a beverage.
Item 12. A method for produicing a food in which a phytosterol and/or a phytostanol is stably dispersed, wherein the composition described in Item 7 or 8 is incorporated in a food raw material.
Item 13. The production method according Item 12, wherein the ratio at which the composition described in Item 7 or 8 is incorporated in a finished food product is 0.1 to 3.2% by weight as the amount of the phytosterol or the phytostanol (the total amount thereof in the case both are contained).
Item 14. The production method according to Item 12 or 13, wherein the food is a beverage.

### IV. Method for Stabilizing Dispersion of a Phytosterol or a Phytostanol in an Aqueous Liquid Product

In addition, the present invention further provides a method for stabilizing dispersion of a phytosterol or a phytostanol in an aqueous liquid product. This invention includes the aspects indicated below.
Item 15. A method for stabilizing dispersion of a phytosterol or a phytostanol in an aqueous liquid product, wherein the composition described in Item 7 or 8 which contains at least one type selected from a phytosterol and a phytostanol is incorporated and dispersed in an aqueous liquid product.
Item 16. The dispersion stabilization method according to Item 15, wherein the aqueous liquid product is a food.
Item 17. The dispersion stabilization method according to 15, wherein the aqueous liquid product is a beverage.

### EFFECT OF INVENTION

According to the production method of the present invention, a phytosterol or phytostanol (comprehensively referred to as "phytosterol/phytostanol" without distinguishing between the two), which was difficult to handle due to poor solubility in water, can be prepared in the form of a composition having superior aqueous dispersibility and aqueous dispersion stability. Since a phytosterol/phytostanol-containing composition obtained according to this production method has superior dispersibility in water and superior stability of that dispersion as described above, it demonstrates satisfactory dispersibility even in the case of applying to an aqueous liquid product containing water in the manner of a beverage, for example, and the occurrence of crystallization, coagulation, ring formation or precipitation of the phytosterol/phytostanol can be significantly inhibited over a long period of time. Consequently, according to the present invention, an aqueous liquid product, and preferably foods, including beverages, can be provided having superior phytosterol/phytostanol dispersion stability.

### BEST MODE FOR CARRYING OUT THE INVENTION

### (1) Production Method of a Phytosterol/phytostanol-Containing Composition and a Composition Obtained with that Method

The production method of a phytosterol/phytostanol-containing composition provided by the present invention comprises at least one of the following steps (1) to (3):
(1) mixing at least one type selected from a pulverized phytosterol and phytostanol with an aqueous raw material containing an emulsifier having an HLB value of 6 to 16;
(2) pulverizing at least one type selected from a phytosterol and a phytostanol followed by mixing with an aqueous raw material containing an emulsifier having an HLB value of 6 to 16; and
(3) mixing at least one type selected from a phytosterol and a phytostanol with an aqueous raw material containing an emulsifier having an HLB value of 6 to 16, and pulverizing the phytosterol and/or phytostanol in the mixture.

In the present invention, a phytosterol includes all phytosterols without limitation, examples of which include natural or synthetic β-sitosterol, stigmasterol, campesterol, brassicasterol, chalinosterol, poriferasterol, clionasterol and isomers thereof These may be used alone or two or more types may be used as a mixture. Naturally-occurring phytosterols can be obtained from vegetable oils such as soybean, rapeseed, cottonseed, wheat germ, rice bran or sesame oil. In general, phytosterols can be acquired commercially, and these commercially available products can be used for the sake of convenience.

In the present invention, a phytostanol includes all natural of synthetic saturated (hydrogenated) or substantially hydrogenated phytosterols and isomers thereof. In the present invention, phytostanols can be used as a substitute for the above-mentioned phytosterols or can be used in combination therewith.

The content of the phytosterol in a composition containing a phytosterol and/or a phytostanol (phytosterol/phytostanol) (the total amount thereof in the case both are contained) is 0.05 to 90% by weight, preferably 0.1 to 45% by weight, and more preferably 1 to 10% by weight. If the content of the phytosterol is too low, effects of the phytosterol/phytostanol cannot be expected, while conversely if the content is too high, the stability of the composition decreases, thereby making this undesirable.

An emulsifier used in the present invention is preferably a component having surface activating effects and an HLB value of 6 to 16, more preferable examples of which include a sucrose fatty acid ester, polyglycerin fatty acid ester and sorbitan fatty acid ester having an HLB value of 10 or more. These emulsifiers are preferably esters of saturated fatty acids such as caproic acid, caprylic acid, capric acid, myristic acid, palmitic acid, lauric acid or stearic acid from the standpoint of not impairing the quality of foods. In the present invention, these emulsifiers may be used alone or two or more types may be used in combination.

The content of the above-mentioned emulsifier is 0.01 to 50% by weight, preferably 1 to 20% by weight and more preferably 1.5 to 10% by weight based on 100% by weight of the composition. If the incorporated amount of the emulsifier is extremely low, emulsification stabilizing effects are not adequately obtained, while if the incorporated amount is extremely high, the unique flavor of the emulsifier has an effect on the food, thereby making this undesirable.

The blending ratio of the phytosterol/phytostanol and the emulsifier used in the method of the present invention can be suitably set and adjusted corresponding to the types of phytosterol and emulsifier used. Preferably, the phytosterol/phytostanol and the emulsifier can be used within a range of the ratio of the phytosterol/phytostanol to the emulsifier of 1:10 to 100:1 (weight ratio), more preferably at a ratio of 1:5 to 50:1 (weight ratio), and even more preferably at a ratio of 1:1 to 10:1 (weight ratio) .

Although there are no limitations on mixing of the phytosterol/phytostanol and the emulsifier in the method of the present invention, mixing is normally carried out by adding the phytosterol/phytostanol to an aqueous raw material containing the emulsifier followed by stirring. Here, examples of the aqueous raw material include water that may contain an arbitrary component (such as an additive or other emulsifier to be described later).

In the production of a phytosterol/phytostanol-containing composition, the production method of the present invention comprises a step in which a pulverized phytosterol/phytostanol is used as a material (above-mentioned step (1)) or a step in which a phytosterol/phytostanol is pulverized (above-mentioned step (2) or step (3)).

In the case of using a preliminarily pulverized phytosterol/phytostanol as a production material of the phytosterol/phytostanol-containing composition, there are no particular limitations on the method used to pulverize the phytosterol/phytostanol, and a pulverized phytosterol/phytostanol is used that has a mean particle diameter of 10 µm or less, preferably 5 µm or less and more preferably 1 µm or less. Furthermore, the mean particle diameter of the phytosterol/phytostanol can be determined by microscopic observation.

Production methods having a step in which a phytosterol/phytostanol is pulverized include (1) a method in which the phytosterol/phytostanol is pulverized followed by mixing with an aqueous raw material containing an emulsifier having an HLB value of 6 to 16, and (2) a method in which an aqueous raw material containing an emulsifier having an HLB value of 6 to 16 is mixed with the phytosterol/phytostanol followed by pulverizing the phytosterol/phytostanol in that mixture. Furthermore, the method of (2) can be applied even in the case of using a phytosterol/phytostanol pulverized in advance to a suitable particle diameter for the phytosterol/phytostanol material, or can be used in combination with the method of (1), namely the method of (2) can be carried out after the method of (1).

Pulverizing the phytosterol/phytostanol can be carried out using an ordinary pulverizing machine without limitation. Furthermore, pulverizing machines consist of wet pulverizing machines and dry pulverizing machines. Pulverizing the phytosterol/phytostanol can be carried out by either of these types of pulverizing (wet or dry) corresponding to the state of the phytosterol/phytostanol, or both types of pulverizing can be used in combination.

Furthermore, a wet pulverizing machine is an apparatus that pulverizes a target substance to be pulverized by mixing in water or an organic solvent and stirring in a container packed with glass, ceramic or other insoluble particles (beads), and a commercially available apparatus may typically be used, examples of which include the Super Apex Mill superfine pulverizing and dispersing machine manufactured by Kotobuki Industries Co., Ltd., the Mitsubishi Diamond Fine Mill manufactured by Mitsubishi Heavy Industries, Ltd. , and the Dyno-Mill manufactured by Shinmaru Enterprises Corporation.

In addition, a dry pulverizing machine is an apparatus that pulverizes a target substance to be pulverized in a dry state, examples of which include those that supply and cause collision of a target substance to be pulverized in a jet air flow generated with compressed air or water vapor sprayed from a high-pressure nozzle to finely pulverize the target substance using the force of collision, and those that cause impact, shear destruction and cutting of a target substance to be pulverized by high-speed rotation of a hammer to finely pulverize the target substance. Examples of these apparatuses include the Turbo Mill, Smoothing Mill and Turbo Counter Mill manufactured by Turbo Kogyo Co., Ltd., the Contraplex and Counter Jet Mill AFG manufactured by Hosokawa Micron Ltd., and the Fine Mill media-agitating fine pulverizing machine with built-in sizer manufactured by Mitsui Mining Co., Ltd.

Although pulverizing conditions can be suitably set and altered corresponding to the shape and state (dry state or moisture-dispersed state) of the phytosterol/phytostanol to be pulverized, the application and type of the phytosterol/phytostanol-containing composition, and the type of the food in which it is to be applied, conditions are preferably such that the mean particle diameter of the pulverized phytosterol/phytostanol is 10 µm or less, preferably 5 µm or less, and more preferably 1 µm or less. Furthermore, the mean particle diameter can be determined by microscopic observation.

A dry pulverizing machine is used preferably in the case of pulverizing the phytosterol/phytostanol prior to mixing with the aqueous raw material containing an emulsifier. On the other hand, a wet pulverizing machine is used preferably in the case of pulverizing a phytosterol/phytostanol in an aqueous raw material containing an emulsifier. There are no particular limitations on the pulverizing conditions in this case provided the conditions allow the particle diameter to be adjusted to the aforementioned mean particle diameter. An example of a method used for wet pulverizing consists of either mixing the phytosterol/phytostanol to be pulverized into an arbitrary solvent such as water or already mixed with an aqueous rawmaterial containing an emulsifier, followed by repeating treatment, consisting of stirring with alumina beads or zirconia beads having a diameter of 0.1 to 1 mm at a rotating speed of 2000 to 4 000 rpm, one to seven times. Furthermore, an example of the rate at which the phytosterol/phytostanol to be pulverized is fed into the pulverizing machine is 50 to 300 g/min.

In addition to the above-mentioned emulsifier (having an HLB value of 6 to 16), one or more types of other commonly known emulsifiers can be used in combination therewith in the production of the phytosterol/phytostanol-containing composition within a range that does not impair the effects of the present invention. Examples of such emulsifiers include glycerin fatty acid esters such as glycerin acetic acid ester, glycerin lactic acid ester, glycerin succinic acid ester, glycerin citric acid ester, glycerin diacetyl tartaric acid ester, polyglycerin fatty acid ester or polyglycerin condensed ricinoleic acid ester; sucrose fatty acid esters, sorbitan fatty acid esters, propylene glycol fatty acid esters, calcium stearoyl lactate, enju saponin, quillaja extract, enzymatically modified lecithin, enzymatically decomposed lecithin, sphingolipid, powdered bile, barley husk extract, enzymatically modified soy bean saponin, soy bean saponin, tomato glucolipid, yucca foam extract, vegetable lecithin, egg yolk lecithin, separated lecithin, tea leaf saponin, beet saponin, polysorbates (polysorbate 20, 40, 60, 65 or 80), gum Arabic, xanthan gum, tragacanth gum, gua gum, gellan gum, locust bean gum, carrageenan, soy bean polysaccharide and other water-soluble polysaccharides.

In addition, additives used in foods, cosmetics, pharmaceuticals and so on may also be added during production of the phytosterol/phytostanol-containing composition within a range that does not impair the effects of the present invention. Examples of such additives that can be arbitrarily added include fragrances; colorants such as β-carotene, annattopigment or purple corn pigment; expiration date extenders such as mustard extract, horseradish extract or kojic acid; preservatives such as milt protein extract, polylysine or sorbic acid; antioxidants such as quercetin, enzymatically modified isoquercitrin or enzymatically modified rutin; thickeners and gelling agents such as carrageenan, xanthan gum, tragacanth gum, gua gum, locust bean gum, alginic acid and alginic acid salts, pectin, tamarind seed gum, ghatti gum, glucomannan, raw starch, processed starch, hemicellulose, water-soluble soy bean polysaccharide, microcrystalline cellulose, fermented cellulose, microfibrous cellulose, carboxymethyl cellulose salts, methyl cellulose, ethyl methyl cellulose, hydroxypropyl cellulose, tar gum, psyllium seed gum, macrophomopsis gum, rhamsan gum, deacylated gellan gum, native gellan gum or gum Arabic; sugars such as sucrose, fructose, glucose, malt syrup, reduced malt syrup, honey, isomerized sugar, inverted sugar, oligosaccharides (such as isomaltooligosaccharide, reduced xylooligosaccharide, reduced gentioligosaccharide, xylooligosaccharide,gentioligosaccharide,nigerooligosaccharide, teandeoligosaccharide and soy bean oligosaccharide), trehalose, sugar-alcohols (such as maltitol, erythritol, sorbitol, palatinit, xylitol and lactitol) or sugar-coupling malt syrup (coupling sugars); high-intensity sweeteners such as aspartame, acesulfame potassium, saccharose, alitame, neotame, licorice extract (glycyrrhizin), saccharin, sodium saccharin, stevia extract or powdered stevia; pH adjusters such as citric acid, fumaric acid or succinic acid; and other extracts, vitamins, minerals, and fat and oils.

A phytosterol/phytostanol-containing composition obtained in this manner has superior aqueous dispersibility and aqueous dispersion stability. The phytosterol/phytostanol-containing composition be in the form of an aqueous solution obtained using the method described above, or it may be in a dry state by using a known drying method- There are no particular limitations on the dry state, and examples of such include a powder prepared using a spray drying method or a freeze-drying method, and granules or tablets obtained by granulation thereof.

### (2) Food Containing a Phytosterol/phytostanol-Containing Composition and Production Method of the Same

The present invention relates to a food stably containing a phytosterol/phytostanol that is prepared by using a phytosterol/phytostanol-containing composition having superior aqueous dispersibility and aqueous dispersion stability obtained by the above-mentioned production method.

There are no particular limitations on foods to which the phytosterol/phytostanol-containing composition of the present invention can be added, and liquid foods containing a water-soluble raw material are preferable. The liquid food is more preferably a beverage containing a water-soluble raw material, examples of which include soy bean, vitamin and mineral drinks, milk, processed milk, milk beverages, lactobacillus beverages, milk products, fermented milk, acidic beverages, neutral beverages, coffee, coffee drinks, coffee-containing soft drinks, tea, tea beverages, tea-containing soft drinks, oolong tea, green tea, whey beverages, carbonated beverages, fruit pulp beverages, fruit juice beverages, fruit juice-containing beverages, fruit juice-containing soft drinks, fruit juice-containing carbonated beverages, beer, cocktails, liquors and other alcoholic beverages, nutritional drinks and other health drinks, flavored water, sports drinks, yoghurt drinks and shakes. Preferable examples of these beverages include fruit juice-related beverages such as fruit juice beverages including orange juice, fruit juice-containing beverages, fruit juice-containing soft drinks and fruit juice-containing carbonated beverages; coffee-related beverages such as coffee, coffee drinks and coffee-containing soft drinks; tea-related beverages such as tea, tea beverages and tea-containing soft drinks, and other preferred beverages.

Moreover, foods targeted by the present invention include not only beverages but also food prepared by containing a water-soluble raw material, examples of which include breads and confections such as cookies and candy; desserts such as jelly, pudding and yoghurt; condiments such as sauce, dressing and gravy; processed foods such as ham; oily processed foods such as margarine, foods spreads and shortening; and other foods such as stew, soup and curry sauce.

In addition, pharmaceuticals containing water-soluble raw materials such as liquid preparations, injection preparations, intravenous drip preparations, syrups, creams, ointments and lotions can also be produced using the present invention.

These foods are produced in accordance with conventionally known methods by using the phytosterol/phytostanol-containing composition of the present invention as one of the raw materials used to produce the food, and then blending with other food raw materials, and special production apparatuses or production conditions are not required.

The ratio of the phytosterol/phytostanol-containing composition of the present invention in a food can be suitably set and adjusted according to the type of food, and although there are no particular limitations thereon, a phytosterol/phytostanol can be contained in a food at a ratio of, for example, 0.1 to 3.2% by weight, preferably 0.2 to 1.6% by weight and more preferably 0.4 to 0.8% by weight. In addition, the phytosterol/phytostanol-containing composition of the present invention can also incorporate an emulsifier (having an HLB value of 6 to 16) in a food so as to be contained at a ratio of 0.02 to 4% by weight, preferably 0.05 to 2% by weight and more preferably 0.1 to 1% by weight.

A food prepared by incorporating the phytosterol/phytostanol-containing composition in this manner significantly inhibits crystallization, coagulation and precipitation of the phytosterol/phytostanol, and allows the phytosterol/phytostanol to be stably dispersed in the food, thereby having superior storage stability.

### (3) Dispersion Stabilization Method of Phytosterol/phytostanol in Aqueous Liquid Product

The present invention provides a method for stabilizing the dispersion of a phytosterol or phytostanol in an aqueous liquid product. This method can be carried out by using the phytosterol/phytostanol-containing composition of the present invention described in (1) above for the phytosterol or phytostanol incorporated in an aqueous product raw material. More specifically, this method can be carried out by incorporating a phytosterol or phytostanol in the form of the phytosterol/phytostanol-containing composition of the present invention in an aqueous liquid product or production raw material thereof followed by stirring and mixing. In addition, emulsification treatment can also be carried out as necessary after having incorporated the phytosterol/phytostanol-containing composition.

There are no particular limitations on the aqueous liquid product targeted by the method of the present invention provided it is water-soluble and in a liquid state, examples of which include pharmaceuticals having the form of, for example, a liquid preparation, injection preparation, intravenous drip preparation, syrup, drink, atomized liquid or lotion; health care products in the form of a liquid such as a mouthwash or toothpaste; cosmetics in the form of a liquid such as a beauty wash, milky lotion, lotion or spray; and foods in the form of a liquid such as various types of beverages and condiments such as sauces, dressings and gravy. Preferably, the aqueous liquid product is a food, and particularly preferably a beverage. The applicable types of beverages are as described in (2) above. Preferable examples of beverages include fruit juice-related beverages such as fruit juice beverages, fruit juice-containing beverages, fruit juice-containing soft drinks and fruit juice-containing carbonated beverages; coffee-related beverages such as coffee, coffee drinks and coffee-containing soft drinks; tea-related beverages such as tea, tea beverages and tea-containing soft drinks, and other preferred beverages.

According to the method of the present invention, crystallization, coagulation and precipitation of a phytosterol/phytostanol contained in aqueous liquid product can be significantly inhibited, the phytosterol/phytostanol can be stably dispersed in the product, and an aqueous liquid product can be provided that has superior storage stability.

### EXAMPLE

Although the following provides a detailed explanation of the contents of the present invention using the following examples, comparative examples and experimental examples, the present invention is not limited thereto. In addition, unless specifically indicated otherwise, "parts" refers to "parts by weight". Furthermore, the phytosterol (powder comprising a mixture of β-sitosterol, stigmasterol, brassicasterol and campesterol; melting point: 140°C) manufactured by Cargill Inc. (USA) was used for the phytosterol in the following examples.

In addition, the mean particle diameter of the phytosterol was first measured using a laser diffraction type of particle size distribution measuring apparatus(laserscatteringsystem;Shimadzu Corp., Japan) followed by confirmation of the measured value by microscopic observation.

### Example 1

Ten parts of polyglycerin stearic acid ester having an HLB value of 12 were added to and dissolved in 300 parts of water. 50 parts of powdered phytosterol (mean particle diameter: 30 µm) were then added thereto followed by dispersing by stirring and subjecting to pulverization treatment with a wet pulverizing machine(Dyno-Mill, Shinmaru Enterprises Corporation; bead diameter: 0. 5 mm, rotating speed: 14 m/s, feeding flow rate: 80 g/min) . Furthermore, the entire production process was carried out at a temperature of 40°C. The mean particle diameter of the phytosterol particles following pulverization treatment contained in the phytosterol-containing composition obtained in this manner was 1 µm or less.

### Comparative Example 1

A phytosterol-containing composition was prepared according to the same treatment and production process as Example 1 with the exception of not carrying out pulverization treatment. The mean particle diameter of the phytosterol particles contained in the phytosterol-containing composition obtained in this manner was 30 µm.

### Comparative Example 2

Twenty-five parts of sucrose stearic acid fatty ester having an HLB value of 11 and 25 parts of sorbitan lauric acid ester having an HLB value of 9 were mixed into 50 parts of a powdered phytosterol (mean particle diameter: 30 µm) followed by dissolving by heating to 140°C and adding to 1000 parts of water based on the description of Patent-ducument 8 (Japanese Published Translation No. 2004-510420 of PCT International Publication). Subsequently, this was then emulsified with a homogenizer to obtain a phytosterol-containing composition in the form of an O/W emulsion having a particle diameter of 0.3 µm.

### Experimental Example 1

The phytosterol-containing compositions of Example 1 and Comparative Examples 1 and 2 obtained in the manner described above were added to orange juice so that the phytosterol content in the final product was 0.8 g/200 ml followed by stirring well. These were then stored for 6 months at room temperature.

As a result, the orange juice to which the phytosterol-containing composition obtained in Example 1 was added did not visibly exhibit any coagulation or precipitation of the phytosterol even after being stored for 6 months at room temperature, was stably dispersed and demonstrated a favorable storage state. In addition, there were also no changes in flavor observed.

On the other hand, although the orange juice to which the phytosterol-containing composition obtained in Comparative Example 1 was added demonstrated a similar degree of flavor as the orange juice to which was added the phytosterol-containing composition of Example 1, ring formation, the formation of an oily film on the liquid surface and precipitation occurred as a result of storing at room temperature. In addition, the orange juice to which was added the phytosterol-containing composition obtained in Comparative Example 2 was observed to form a ring and form an oily film on the liquid surface as a result of storing at roomtemperature. In addition, this orange juice gave off a strong emulsifier odor and had inferior flavor.

### Example 2

A pre-powdered phytosterol (mean particle diameter: 30 µm) was pulverized to a mean particle diameter of 10 µm or less using a dry pulverizing machine (Nara Machinery Co., Ltd., Jiyu Mill, screen diameter: 0.5 mm).

Five parts of sucrose fatty acid ester having an HLB value of 12, 5 parts of polyglycerin stearic acid ester having an HLB value of 12 and 40 parts of dextrin were heated and dissolved in 300 parts of water. The above-mentioned pulverized phytosterol (mean particle diameter: 6 µm) was added to and dispersed therein followed by subjecting to pulverization treatment using a wet pulverizing machine (Dyno-Mill, Shinmaru Enterprises Corporation). The mean particle diameter of the phytosterol at this time was 1 µm or less.

The resulting aqueous solution was spray-dried with a spray dryer to prepare a powdered phytosterol-containing composition.

### Comparative Example 3

A liquid phytosterol-containing composition (phytosterol mean particle diameter: 30 µm) was prepared according to the same treatment and same production process as Example 2 with the exception of not carrying out pulverization treatment with a wet pulverizing machine (Dyno-Mill). Next, the resulting aqueous solution was spray-dried with a spray dryer to prepare a powdered phytosterol-containing composition.

### Experimental Example 2

The phytosterol-containing compositions obtained in Example 2 and Comparative Example 3 were added to apple juice so that the phytosterol content in the final product was 0.8 g/200 ml followed by stirring well. These were then stored for 6 months at room temperature.

As a result, the apple juice to which the phytosterol-containing composition obtained in Example 2 was added did not exhibit any ring formation or precipitation even after being stored for 6 months at room temperature, the phytosterol was stably dispersed, and the apple juice demonstrated favorable storage stability. In addition, there were also no changes in flavor observed. On the other hand, the apple juice to which the phytosterol-containing composition obtained in Comparative Example 3 was added demonstrated ring formation, the formation of an oily film on the liquid surface and precipitation after being stored for 6 months, and storage stability was poor. Although the flavor was similar to the apple juice to which was added the phytosterol-containing composition of Example 2, a gritty sensation was perceived when contained in the mouth.

## Claims

1. A method for producing a composition containing at least one type selected from a phytosterol and a phytostanol, comprising at least one of the following steps (1) to (3):
(1) mixing at least one type selected from a pulverized phytosterol and phytostanol with an aqueous raw material containing an emulsifier having an HLB value of 6 to 16;
(2) pulverizing at least one type selected from a phytosterol and a phytostanol followed by mixing with an aqueous raw material containing an emulsifier having an HLB value of 6 to 16; and
(3) mixing at least one type selected from a phytosterol and a phytostanol with an aqueous raw material containing an emulsifier having an HLB value of 6 to 16, and pulverizing the phytosterol and/or phytostanol in the mixture.

2. The method according to claim 1, wherein the mean particle diameter of the pulverized phytosterol and/or phytostanol is 10 µm or less.

3. The method according to claim 1, wherein the composition containing at least one type selected from a phytosterol and a phytostanol contains the phytosterol or the phytostanol at a ratio of 0.05 to 90% by weight.

4. The method according to claim 1, wherein the emulsifier is at least one type selected from a sucrose fatty acid ester, a polyglycerin fatty acid ester and a sorbitan fatty acid ester having an HLB value of 10 or more.

5. The method according to claim 1, wherein the composition containing at least one type selected from a phytosterol and a phytostanol contains an emulsifier at a ratio of 0.01 to 50% by weight.

6. The method according to claim 1, wherein the composition containing at least one type selected from a phytosterol and a phytostanol contains the phytosterol or the phytostanol and an emulsifier at a weight ratio of 1:10 to 100:1.

7. A composition containing at least one type selected from a phytosterol and a phytostanol obtained by the method according to claim 1.

8. The composition according to claim 7, wherein the mean particle diameter of the phytosterol and/or phytostanol is 10 µm or less.

9. A food containing the composition according to claim 7.

10. The food according to claim 9, wherein the composition according to claim 7 is contained at the ratio of 0.1 to 3.2% by weight as the amount of the phytosterol or the phytostanol (the total amount thereof in the case both are contained).

11. The food according to claim 9, which is a beverage.

12. A method for producing a food in which a phytosterol and/or a phytostanol is stably dispersed, wherein the composition according to claim 7 is incorporated in a food raw material.

13. The method according to claim 12, wherein the ratio at which the composition according to claim 7 is incorporated in a finished food product is 0.1 to 3.2% by weight as the amount of the phytosterol or the phytostanol (the total amount thereof in the case both are contained).

14. The method according to claim 12, wherein the food is a beverage.

15. A method for stabilizing dispersion of a phytosterol or a phytostanol in an aqueous liquid product, wherein the composition according to claim 1 which contains at least one type selected from a phytosterol and a phytostanol is incorporated and dispersed in an aqueous liquid product.

16. The dispersion stabilization method according to claim 15, wherein the aqueous liquid product is a food.

17. The dispersion stabilization method according to claim 15, wherein the aqueous liquid product is a beverage.
